# EUROPEAN PATENT SPECIFICATION

(11) **EP 2 433 102 B1**
(45) Date of publication and mention of the grant of the patent: **04.05.2016**
(21) Application number: 10778194.0
(22) Date of filing: 17.05.2010
(51) Int. Cl.: G01J 3/44, G01N 21/65

(54) **LARGE AREA SCANNING APPARATUS FOR ANALYTE QUANTIFICATION BY SURFACE ENHANCED RAMAN SPECTROSCOPY**
WEITBEREICHS-SCANVORRICHTUNG ZUR ANALYTQUANTIFIZIERUNG DURCH OBERFLÄCHENVERSTÄRKTE RAMAN-SPEKTROSKOPIE
APPAREIL DE BALAYAGE DE GRANDE SURFACE POUR LA QUANTIFICATION D'ANALYTES PAR SPECTROSCOPIE RAMAN EXALTÉE EN SURFACE

(30) Priority: 18.05.2009 US 467440
(43) Date of publication of application: 28.03.2012
(73) Proprietor: Bruker Optik GmbH, 76275 Ettlingen (DE); EMD Millipore Corporation, Billerica, MA 01821 (US)
(72) Inventor: TAGUE, Thomas, Richmond, NH 03470 (US); KOPACIEWICZ, William, West Newbury, MA 01985 (US); DAVISSON, Vincent, West Lafayette, IN 47906 (US); CHERNOKALSKAYA, Elena, Lexington, MA 02420 (US); RIDER, Timothy, Exeter, NH 03833 (US); HINOJOS, Cruz, Spring, TX 77386 (US); ZHAO, Jun, Conroe, TX 77384 (US)
(74) Representative: Kohler Schmid Möbus Patentanwälte
(86) International application number: PCT/US2010/035076
(87) International publication number: WO 2010/135226

(56) References cited:
- WO-A2-2007/090058
- JP-A- 2002 257 721
- JP-A- 2006 258 990
- JP-A- 2007 135 989
- US-A- 5 946 090
- US-A1- 2004 008 522
- US-A1- 2005 048 581
- US-A1- 2006 055 919
- US-A1- 2007 127 022
- US-A1- 2008 316 466
- US-B1- 6 281 971
- US-B1- 6 351 306

## Description

### BACKGROUND

The protein immunoblot (commonly called a "Western blot") is a widely used method for the study of proteins. The information gained from the Western blot method includes individual protein abundance and/or expression and individual protein size when protein size standards are incorporated in the method. The specificity inherent to the method is produced by the use of antibodies for protein identification and detection (immunodetection). The utility of the Western blot has made it a commonly used method in biology and biochemistry labs throughout academia and industry and has spurred innovative improvements in the method.

While many variations exist, the standard Western blot method includes the steps of separating proteins from a heterogeneous protein mixture according to protein size and charge by gel electrophoresis, transferring the separated proteins to a solid support or blot, which is usually composed of nitrocellulose or polyvinylidine fluoride, binding the protein of interest to an antibody, and detecting a signal. The greatest variation in the method occurs at the detection step. Most commonly, a detectable signal is generated by attaching a second antibody (referred to as a secondary antibody) to the protein-specific primary antibody. The secondary antibody is conjugated to a signal-producing moiety either before or after attachment and the moiety generates the detected signal.

Another commonly-used clinical and research method for the identification and/or quantitation of a wide range of molecules is enzyme linked immunosorbant assay (ELISA). The types of molecules that can be assayed with this method include, but are not limited to: allergens, drugs, small molecules and peptides, and proteins. As such, common clinical applications of the method include diagnosis of specific allergies or infection as well as detection of drugs or steroids. The wide use of the assay is indicative of the utility and robustness of the method.

The purpose of the ELISA method is to detect and/or quantify an antibody or an antigen within a sample. There are two principal method variations. The standard method involves pre-absorbing a sample-containing antigen onto a plastic well plate bottom. The antigen is specifically immunolabeled with a primary antibody and detected by a secondary antibody specific to the primary antibody. As with the Western blot, the secondary antibody is typically conjugated to a signal-producing moiety. The second method is more specific and involves the pre-absorbing of an antigen specific, primary antibody to the well plate bottom, addition of the sample-containing antigen, and antigen immunolabeling and detection as described for the standard method. The increased specificity is imparted by the use of two primary antibodies for labeling. Other variations to the method exist, but all rely on the use of antibodies to label and detect molecules of interest.

The most common types of signal produced by the Western blot and ELISA methods are chemiluminescence (chemically produced light), color, radioactivity, and fluorescence; of these, chemiluminescence is the most prevalent. To produce chemiluminescence in a Western blot or an ELISA plate, the secondary antibody is most commonly conjugated to the enzyme horseradish peroxidase. The addition of the enzyme's substrate then produces a chemiluminescent product. In the case of a Western blot, the light produced is detected by light-sensitive film or by charge coupled detector cameras and the information is analyzed to determine protein identity. In the case of ELISA, light signals are usually detected and quantified with the appropriate well-plate scanner. It is also possible to enhance the chemiluminescence with various proprietary methods. Enhanced chemiluminescence (ECL) can be highly sensitive, allowing nanogram amounts of protein to be detected within minutes.

However, ECL and the other traditionally used signals each have drawbacks. For example, the use of radioactivity requires adherence to safe use and disposal guidelines. Colorimetry and ECL each require the mixing and/or the incubation of the blots and plates with enzyme substrates thereby increasing the processing time and the colorimetric and ECL signals are generated by an enzymatic reaction and, thus, are only indirect indicators of protein amount. Colorimetry also has a narrower dynamic range than the other signals. Further, each signal producer has a finite lifetime and, therefore, a limited ability to be detected. For these reasons, there is a demand for a simplified, highly sensitive, and direct platform for detection of proteins.

Raman spectroscopy is a spectroscopic technique used to study vibrational, rotational, and other low-frequency modes of materials. The technique relies on inelastic scattering of monochromatic light in the visible, near infrared, or near ultraviolet range. The excitation light interacts with phonons or other excitations in the material, resulting in a shift in the energy of the light photons, from which Raman shift information about the phonon modes in the system can be derived. A Raman spectrum contains many different peaks. Each peak corresponds to the energy of the vibration of a chemical bond in a molecule. Therefore, a Raman spectrum can be nearly a unique fingerprint of the molecule and it would be desirable to use Raman spectroscopy to analyze biomolecular patterns in large area samples, such as Western blot and ELISA samples.

However, Raman spectrometers have not been used to analyze large area samples for two reasons. The first is measurement sensitivity, which is determined by factors attributable to both the sample and the instrument itself, including the Raman scattering cross section of the sample, the optical power of the excitation light, the solid angle of Raman signal collection, and the quantum efficiency and noise characteristics of the Raman signal detector. For biomolecules of the type found in Western blot and ELISA samples, the Raman scattering cross section is small and therefore, the sensitivity is low.

The second reason is that Raman spectrometers are typically designed to use the excellent focusing capability of the laser beam and a microscope system to produce a high spatial resolution, which is a key advantage of Raman spectroscopy. Spatial resolutions on the order of approximately one micrometer can be routinely achieved on a conventional microscope based system. Many such Raman microscopy systems are commercially available; one such system is disclosed in a U.S. Patent No. 7,102,746 B2.

Unfortunately, in order to analyze an entire sample having a size on the order of 100 x 100 mm with a spectrometer having a spatial resolution of one micrometer, 10,000,000,000 spectra would have to be acquired. The fastest multichannel detectors used in Raman spectroscopy can only acquire less than 1000 spectra per second and consequently, this process would take nearly four months. A side effect associated with such a high resolution is that very low laser power must be used, because photo and thermal damage that can result from the highly focused laser beam. Everything else being equal, the signal strength of the acquired Raman spectrum is proportional to the total excitation light power. The weaker Raman signal that results from a low excitation power often has to be compensated by increasing the spectral acquisition time, which further reduces the measurement speed.

In a Western blot sample, such as a cell lysate, , there are typically on the order of 10 lanes, each lane occupying a width of several millimeters and containing more than 1000 distinct protein bands. Since the proteins are separated along the length of the lanes, the spatial resolution along the width of the lanes is not critical, and can be as wide as the lane itself as long as it less than the distance between the lanes. The spatial resolution along the length of the lane can be set between 0.1 to 1 mm. If the spatial resolution is set to be 0.5 mm wide and 0.2 mm long, the number of spectra required to cover a 100x100 mm area is reduced to 100,000. At 100 spectra per second sampling rate, the analysis would only take 17 minutes, which is manageable.

Similarly, ELISA samples are typically prepared in microtiter plates (8 X 12 cm); one standard format has 96 wells, each well having a circular area with a diameter of 5.5 mm. The goal of the analysis is to determine the total amount of the species of interest, regardless of its spatial distribution inside the well. Thus the spatial resolution can be set even lower than Western blot, which will greatly improve analysis speed.

Therefore, one straightforward method of using a Raman spectrometer to analyze Western blots is to decrease the spatial resolution of the spectrometer so that it is on the order of, or larger than, 0.1 square millimeter. However, a property of optical systems called *etendue* makes it difficult to obtain such a large sampling area. The *etendue* constant of an optical system is the product of the solid angle of collection and the illumination area. Accordingly, simply illuminating a large sample area would result in an unacceptably small solid angle of collection. Opening the entrance slit of the spectral analyzer wider to increase the sampling area with a very small solid angle of collection will degrade the spectral resolution; therefore this solution is not advisable either. Consequently there is a need for an apparatus and method to analyze larger surface area samples, such as Western blot or 96 well ELISA via Raman spectroscopy.

JP 2006-258990A discloses a Raman-spectroscopy microscope comprising a cylindrical lens converting a laser beam from source into a line-like light beam and a galvanometer mirror which scans the line-like light beam over a sample. The Raman microscope of the optical microscope has a laser which emits a monochromatic laser beam which is converted by cylindrical lens to an incident light line in the Y direction. An iris diaphragm has a slit-shaped opening which corresponds to the direction of the line-like incident light. A galvanometer mirror reflects the light in the direction of lens so as to scan the incident line of light. That is, a galvanometer mirror is a scanning means to scan the light beam converted in the shape of a line.

### SUMMARY

In accordance with the principles of the disclosure the sensitivity of the measurement is increased by conjugating secondary antibodies used in the Western blot and ELISA methods to Raman labels. The resulting blot or well plate is analyzed with a Raman system that has a large sampling area and acceptable solid angles of collection. More specifically, the Raman system generates an effectively line-shaped illumination pattern and scans the sample in the direction perpendicular to the line while the signal is accumulating on the detector. The sampling area is therefore a rectangle defined by the length of the illumination and the distance traveled by the sample within the duration of signal accumulation on the detector.

In one embodiment, the line shaped illumination pattern is generated by a cylindrical or Powell lens inserted in the excitation light beam path.

In another embodiment, the line shaped illumination pattern is generated by a fast scanning optic inserted in the excitation light beam path. Although at any instant the illumination pattern is a focused round spot, the rapid scanning of the beam effectively creates a line illumination pattern.

In yet another embodiment, the line shaped illumination pattern is generated by a fast scanning optic inserted in the combined excitation beam and Raman signal beam path.

In still another embodiment, the sample to be analyzed is mounted on a stage that can be mechanically translated at high speed under the excitation light beam.

### BRIEF DESCRIPTION OF THE DRAWINGS

Figure 1 is a block schematic diagram of a scanning Raman spectrometer constructed in accordance with the principles of the invention that uses an optical device in the excitation beam path to produce a line-shaped illumination pattern.
Figure 2 is a picture of a Western blot sample showing an illustrative scanning pattern.
Figure 3 is a picture of the Western blot sample shown in Figure 2 showing an alternative scanning pattern.
Figure 4 is a block schematic diagram of an alternative embodiment of a scanning Raman spectrometer that uses a Galvano mirror optical device in the excitation beam path to produce a line-shaped illumination pattern.
Figure 5 is a block schematic diagram of another embodiment of a scanning Raman spectrometer that uses a Galvano mirror optical device in the combined excitation - Raman beam path to produce a line-shaped illumination pattern.
Figure 6 is a flowchart illustrating a process for preparing a Western blot sample for scanning and detection.
Figure 7 is a flowchart illustrating a process for preparing an ELISA sample for scanning and detection.
Figure 8 is a flowchart illustrating a process for preparing a microarray sample for scanning and detection.

### DETAILED DESCRIPTION

A Raman spectrometer contains at least four basic modules: a source for generating an excitation beam, a spectral analyzer, a detector, and optics for focusing the excitation beam onto the sample, for collecting the Raman signal from the sample, and for focusing the Raman signal into the spectral analyzer. Modern instruments use lasers exclusively for an excitation source, with a wide selection of wavelengths ranging from ultraviolet to near infrared. The excitation beam is directed at the sample and produces a signal beam having both a strong elastically-scattered, or Rayleigh, component at the excitation beam wavelength and an inelastically scattered, or Raman, component.

The spectral analyzer decomposes the Raman component into its many constituent frequencies and can take one of several forms. A dispersive analyzer uses a wavelength dispersing element, such as a grating or a prism, to separate the different wavelengths in space. An FT-Raman analyzer utilizes an interferometer to generate an interferogram from the signal. The interferogram is then transformed into frequencies via a mathematical procedure. A another form of analyzer uses a tunable filter, such as an acousto-optic tunable filter (AOFT), or a liquid crystal tunable filter (LCTF) to pass one frequency at a time.

The different frequencies are then applied to a detector. Detectors commonly used for Raman spectroscopy include single detectors (such as photomultiplier tubes for monochromators working in the visible region and GaAs or cooled Ge detectors for FT-Raman spectroscopy using near infrared excitation wavelength), and multichannel sensors (such as charge coupled devices working in the visible and ultraviolet region, and InGaAs array detectors in the near infrared region, both used with dispersive spectral analyzers).

Other critical optics for Raman spectroscopy include laser band pass filters for purifying a monochromatic source, and laser rejection filters for removing the overwhelmingly strong Rayleigh component prior to sending the Raman signal to the spectral analyzer. In the most common configuration, which is called the back-scattering or epi configuration, the same optics perform both functions of focusing the excitation beam onto, and collecting the Raman signal from, the sample.

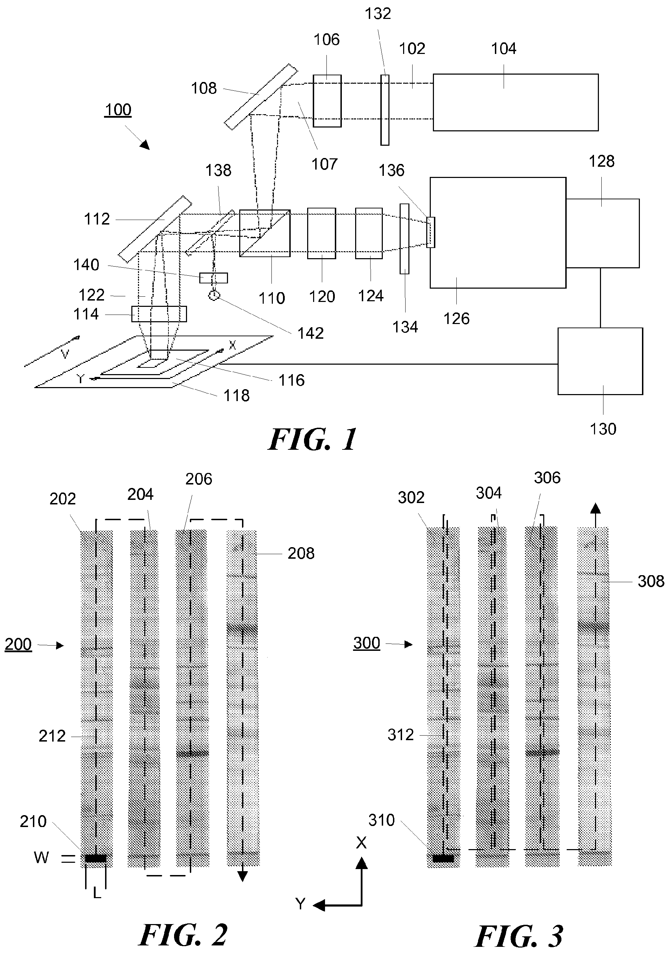

Figure 1 is a schematic diagram of one embodiment of a Raman spectroscopic apparatus 100 constructed in accordance with the principles of the invention for rapid scanning of a large area sample, such as a Western blot or ELISA plate. An optical element is inserted in the excitation beam path to form a line focused illumination pattern on the sample. More particularly, a substantially collimated excitation beam 102 from the laser source 104 is focused by an optical element 106, which may be a cylindrical lens or a Powell lens, in only one dimension.

The line focused illumination pattern 107 is then directed by a mirror 108 toward a beam combining module 110. Other optics, not shown in Figure 1, such as a beam expander, can be inserted in the excitation beam to control the size of the line shaped illumination pattern. The beam combining module 110 reflects the laser excitation beam toward another re-directing mirror 112, which reflects the beam toward an objective lens 114.

The objective lens 114 focuses the excitation beam on the sample 116 into a narrow line of length L along the Y direction at the focal plane of the lens. The Raman signal beam 122 is collected by the same objective lens 114, reflected by mirror 112, passes through the beam combining module 110, which also reflects the majority of the Rayleigh component, then passes through one or more Rayleigh rejection filters 120, and is focused by a lens 124 onto the entrance slit 136 of the spectral analyzer 126. The orientations of optical element 106 and the analyzer 126 are such that the length of slit 136 is parallel to the image of the line-shaped illumination pattern.

The analyzer 126 separates the Raman signal into different wavelengths and projects the spectrum onto a multichannel detector 128. Any one of several types of spectrographs that have been used with multichannel detectors for Raman spectroscopy can be used for spectral analyzer 126. A standard Czerny-Turner spectrograph is disclosed in a U.S. Patent No. 7,102,746 B2. An axial reflectance spectrograph using lenses with a plane reflective grating is disclosed in a U.S. Patent Nos. 6,281,971 B1; 6,353,476 B1 and 6,636,305 B2. Other types of spectrographs using Volume Holographic Transmission gratings, concave or convex gratings are also well-known.

Multichannel detectors commonly used in Raman spectrographs include 2-D Charge Coupled Device (CCD) cameras, linear CCD arrays, Electron Multiplying CCDs (EMCCD), Avalanche Diode Arrays, linear and 2-D InGaAs arrays. The Raman signal accumulates on the detector 128 for a duration of time T, during which time the stage 118 moves the sample in the X direction at speed V. Thus the sampled area is a rectangle with a length L and a width W equal to the distance traveled by the stage during time T, which is speed V multiplied by the time T.

The stage 118 and the detector are controlled by the computer 130 such that the spectra acquired can be correlated with the sample positions. To generate a Raman spectral map of the sample, the sample stage 118 can be scanned in a raster pattern while the detector acquires Raman spectra, each pixel in the map corresponds to a rectangular area of length L and width W. In case the sample is a Western blot, the lanes of the Western blot are preferably aligned along the X axis of the stage. Optionally, one or more shutters 132 and 134 may be inserted in the laser beam and the Raman beam to control acquisition of the spectrum.

Figures 2 and 3 illustrate two sample scanning patterns for use with Western blot samples. The Western blot sample 200 has four lanes 202-208. In this example, the sampling area 210 with length L and width W travels in a direction indicated by arrow 212 up one lane and down the other. Figure 3 shows an alternative scanning pattern. Here the Western blot sample 300 also has four lanes 302-308. The sampling area 319 travel in the direction of arrow 310 up each lane and then returning to the start of the lane before moving to the next lane.

Once the Raman spectra are acquired, they can be processed with software to generate qualitative and quantitative information regarding the chemical composition at any location of the sample. Combining this information with the positional information, a compositional map can be obtained of the sample.

The purpose of creating a line-focused illumination pattern is to create a relatively large length L of the rectangular area, thus increasing the speed of mapping at the sacrifice of reduced spatial resolution. Without the cylindrical or Powell lens 106, the laser would be focused into a much smaller spot, and it would take much longer time to map the same area. Another benefit of using a line-focused illumination pattern is that the power density on the sample is greatly reduced, and higher total power can be used to yield stronger signal without damaging the sample, as result, the signal accumulation time T for each spectrum can be significantly reduced, and scanning speed V increased, thus achieving even faster mapping speed.

The large sampling area with a large solid angle of collection does not violate the optical principle of a constant *etendue* for the optical system because the sampling area is not the same as the illumination area which factors into the *etendue* parameter. At any instant, the illumination area is still very small, which allows a large solid angle to be used. The apparent large sampling area is caused by the motion of the sample and/or the excitation beam, and produces the benefits, among others, that include, a much faster mapping speed for samples that do not require higher spatial resolution, and reduced instrument size and cost.

In this embodiment, the spatial resolution in the X and Y directions can be adjusted independently. The spatial resolution in the Y direction is the length of the laser line L, and can be adjusted by changing the effective focal length of the lens 106 or the objective lens 114. It would be known to those skilled in the art that, by combining a group of optical elements, a variable focal length lens can be constructed. The spatial resolution in the X direction is width W, which can be adjusted by varying the scanning speed V of the stage in the X direction, or the spectral acquisition time T. All these parameters can be kept constant to generate a map of constant spatial resolution, or made to vary to result in a map of variable spatial resolution.

An internal wavelength calibration unit can be built into the instrument to facilitate automatic periodic calibration. This can be achieved by means of a mode mirror 138. During sample measurement, this mode mirror is moved out of the beam path. In calibration mode, this mirror is moved into the beam path so that the laser beam is directed toward a lens 140 and focused onto a reference sample 142 made of a reference Raman material. A reference Raman material has a number of Raman peaks whose Raman shifts in wavenumbers (cm⁻¹) are accurately known. An example of this is 4-acetomidophen, the active ingredient of the drug Tylenol. The Raman spectrum of the reference sample 142 is then acquired, and used to calibrate the wavelength into standard Raman shift. Alternatively, if the excitation beam from source 104 has a known wavelength, the reference sample 142 can be replaced with an atomic emission lamp, such as a neon arc lamp. Wavelengths of many of the spectral lines of such an emission lamp are accurately know, and can be used to calibrate the instrument into absolute wavelengths and therefore absolute wavenumbers. The absolute wavenumber is then subtracted from the wavenumber of the excitation to yield the Raman shift in wavenumbers (cm⁻¹).

Alternatively, the mode mirror 138 can be replaced with a stationary beam splitter, which has a high transmittance and a low reflectance. In this case, the emission lamp 142 can be turned on and off electronically, independent of the excitation beam. This reduces the number of moving parts in the instrument and may speed up the calibration process.

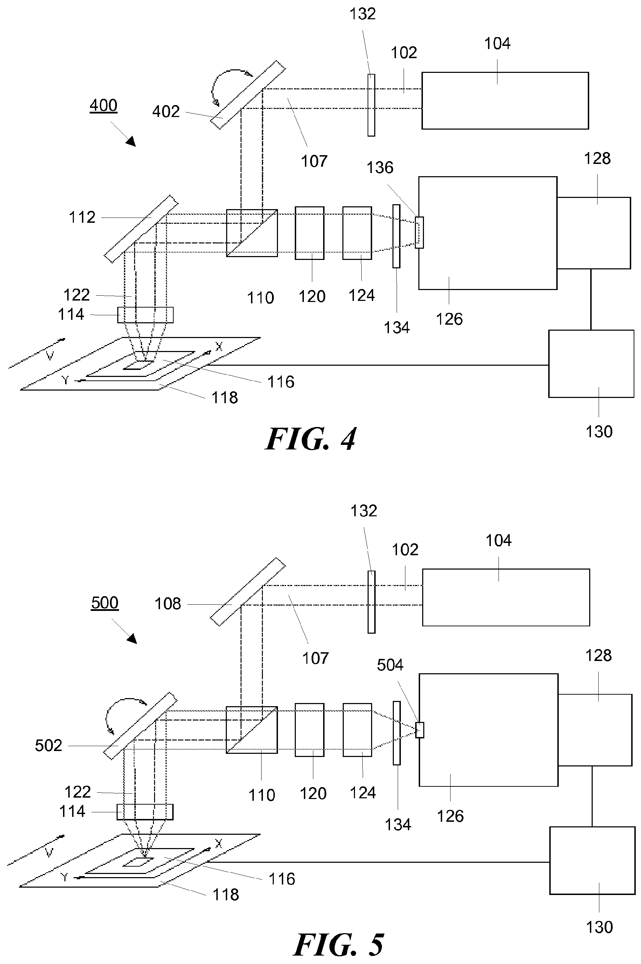

Figure 4 shows the schematic of another embodiment of the Raman spectroscopic apparatus 400. In Figure 4, elements that correspond to elements in Figure 1 have been given the same numeral designations and will not be described further in detail. In this embodiment, the line-shaped illumination pattern is generated by rapidly scanning a Galvano mirror 402. At any instant, the laser beam is focused into a spot. However, the rapid scanning of the mirror effectively creates a line-shaped pattern. The length of this line L is determined by the angular swing amplitude of mirror 402 and the focal length of the objective lens 114. When the stage 118 travels in the X direction, an effective uniform illumination is obtained over a rectangular area of length L and width W, where W has the same meaning as in Figure 1.

Figure 5 shows the schematic of another embodiment of the Raman spectroscopic apparatus 300. In Figure 5, elements that correspond to elements in Figures 1 and 4 have been given the same numeral designations and will not be described further in detail. Here the mirror 108 is stationary, while the line shaped illumination pattern is generated by rapidly scanning the Galvano mirror 502. As with the apparatus shown in Figure 4, this scanning effectively creates a line-shaped illumination. The difference is that in this configuration, mirror 502 is in the combined excitation beam - Raman beam path, thus mirror 502 also scans the Raman beam, such that the Raman beam is always focused onto the slit 504 at the same position. This allows a much shorter slit and detector to be used. A smaller detector such as linear CCD array costs substantially less than a larger detector of the same type, such as a two-dimensional CCD array detector. As with the embodiment shown in Figure 1, internal wavelength calibration components 138, 140 and 142 can be incorporated into the embodiments shown in Figures 4 and 5.

As previously mentioned, another problem with Raman measurements is their low sensitivity. However, the Raman scattering cross section of the sample and thus the sensitivity of the measurement can be boosted dramatically by several enhancement techniques. In a technique called Resonance Raman Spectroscopy (RRS), the excitation wavelength is chosen to match an electronic transition in a molecule of interest. Thus RRS can be employed to selectively enhance the characteristic Raman signature of a target molecule in a complex matrix of molecules. RRS is particularly useful for large biomolecules that incorporate resonance chromophores in their structures because the excitation wavelength can be use to excite a transition in the chromophore. When a molecule of interest lacks such resonance chromophores, the molecule can be attached to molecular tags that do produce resonance enhancement, similar to fluorescence tagging widely used in biochemical analysis.

In another enhanced Raman technique called Surface Enhanced Raman Spectroscopy (SERS), the weak Raman scattering intensity is greatly strengthened (by a factor of many orders of magnitude as compared to the intensity obtained from the same number of molecules in solution or in the gas phase) by attaching the molecules which produce the inelastic scattering to Raman labels. As used herein a "Raman label" is any entity that imparts a traceable Raman signal to a molecule. Examples include dyes, metal structures of nanoscale size (nanoparticles), dye-nanoparticle (enhancer) constructs and variants thereof. As used herein "nanoparticles" are any of a class of typically metallic colloidal substances in the range of 2 to 200 nm that enhance Raman spectral signals.

One method of using SERS to detect proteins involves tagging the proteins with Raman active dyes. As used herein a "dye" is any of a class of organic substances that have usable optical properties and generate Raman spectra. In one embodiment, the dyes are isotope-substituted dyes, each of which has a distinct SERS spectral signature. This method is described in detail in PCT publication number WO 2006/037036 A2. The isotope coded SERS dyes offer unique Raman spectral signatures, high sensitivity and unlike fluorescent labels, the SERS dyes do not quench, enabling long detection times and maximizing sensitivity. In this method, sample proteins are labeled with the SERS dyes, separated by electrophoresis and subjected to Raman analysis.

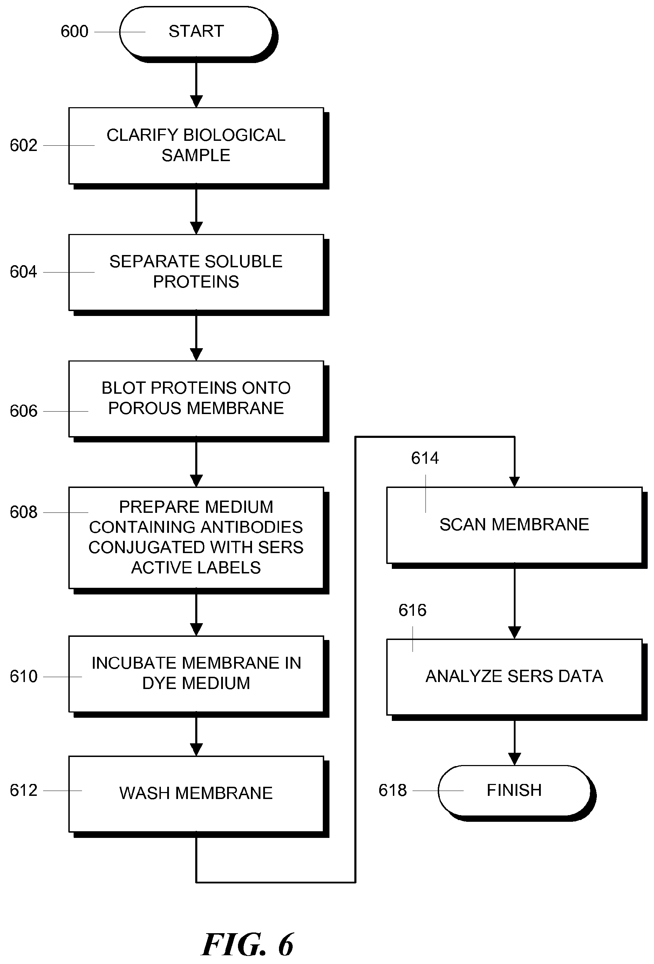

Figure 6 is a flowchart showing the steps in preparing a sample for a Western blot protein immunodetection and quantification using SERS detection with the inventive scanning system. This process begins in step 600 and proceeds to step 602 where the biological sample is clarified, for example, by centrifugation. Next, in step 604, soluble proteins are then physically separated, for example, by using gel electrophoresis. In step 606, the separated proteins within the electrophoresis gel are then transferred or blotted onto a porous membrane while maintaining their relative positions. Then, in step 608, a detection reagent containing antibodies labeled with Raman active dyes is prepared. As used herein a "detection reagent" is any of a class of substances that, when associated with a sample, either specifically or non-specifically impart some type of unique detectable signal to one or more analytes. In general, antibodies which selectively capture the proteins of interest are used. Next, in step 610, the membrane on which the proteins have been blotted is immersed in the detection reagent and incubated to allow the antibodies to detect and quantify the individual proteins. The membrane is scanned in step 614. The resulting SERS data is analyzed based on physical positioning in step 616 to generate the result and the process ends in step 618.

The inventive system can be used to detect multiple proteins. The ability to detect and measure two or more proteins at the same time is called "multiplexing". The detection is carried out as described above with respect to the Western blot detection, only that multiple antibodies conjugated to distinctively different Raman labels are used to detect multiple antigens on the membrane simultaneously. The membrane is scanned using the Raman spectrometer system described above, signals from bound Raman labeled antibodies are detected and amounts of respective proteins are calculated.

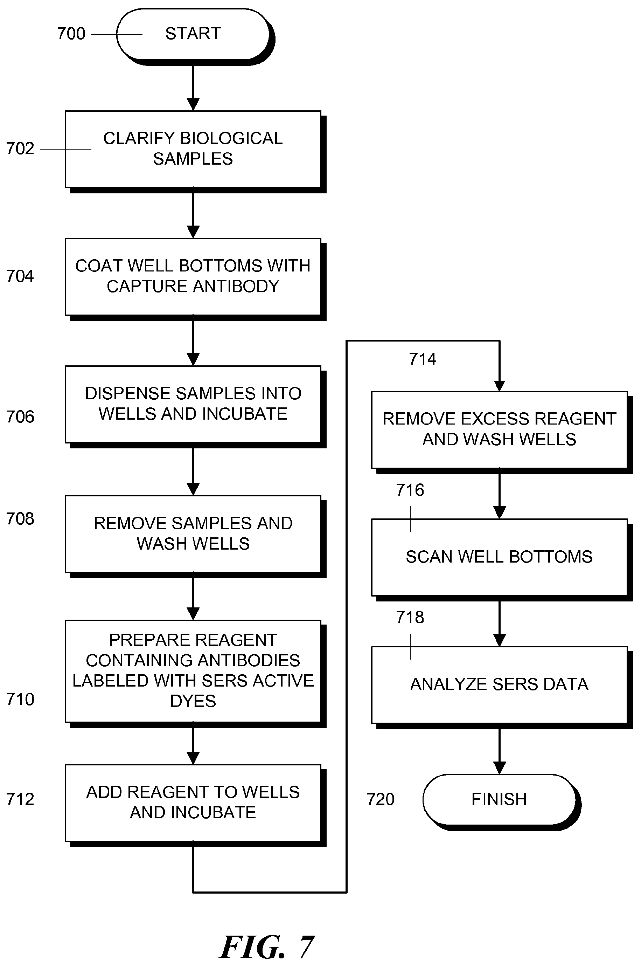

The process for preparing a sample for an ELISA protein immunodetection and quantification using SERS detection with the inventive scanning system is similar to the Western blot process. The ELISA process is typically performed in a multi-well plate with a porous membrane or a solid plastic bottom and an illustrative example is shown in Figure 7. The process begins in step 700 and proceeds to step 702 where the biological sample is clarified, for example, by centrifugation. Next, in step 704, the well bottoms are coated with a capture antibody.

In step 706, the clarified biological samples are dispensed into the wells and incubated for a predetermined time period to allow for analyte capture. Then, in step 708, the samples are removed from the wells and the wells are washed. A detection reagent containing a biospecific binder labeled with Raman active dyes is prepared in step 710. As used herein a "biospecific binder" is any of a class of biological (for example, proteins, nucleic acids, etc.) or biosynthetic molecules (for example, enzyme inhibitors) that exhibit highly specific binding properties towards an analyte. An example might be detection antibodies. The detection reagent is added to the wells in step 712. After a predetermined period of time to allow for detection, in step 714, excess detection reagent is removed and the wells are again washed. In step 716, the well bottoms are scanned to determine the amount of analyte by detecting a SERS signal. The SERS data is analyzed in step 718 and the process finishes in step 720.

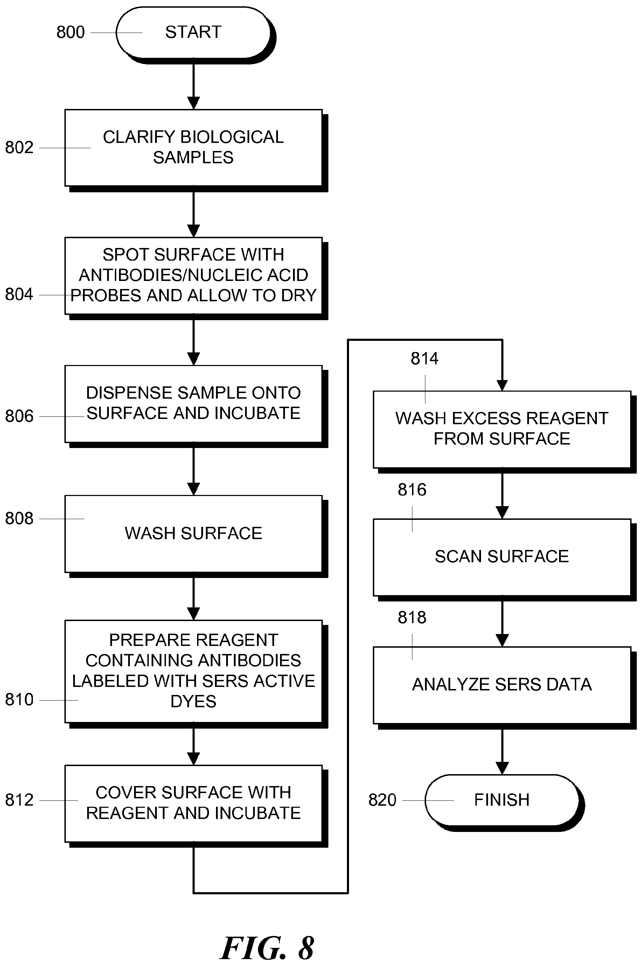

In another embodiment, proteins on a microarray can be detected and quantified. This embodiment is very similar to the multi-well plate embodiment discussed with respect to Figure 7 albeit at a smaller scale. This process, shown in Figure 8, begins in step 800 and proceeds to step 802 where the sample is again clarified by centrifugation. In step 804, tiny amounts (< 0.1 microliter) of a plurality of capture antibodies or other biospecific reagent (for example, a nucleic acid probe) are spotted in an addressable way on a flat solid surface, such as a glass slide, and allowed to dry. The clarified sample is then dispensed onto the surface in step 806 and given time for analyte capture. Next, in step 808, the surface is washed. In step 810, the detection reagent containing the biospecific binder conjugated to Raman labels is prepared and, in step 812, used to cover the surface. After a period of time to allow for detection, excess detection reagent is washed away in step 814. Next, in step 816, the biospecific reagent spots are scanned to determine the amount of analyte via the SERS signal. Finally, the SERS data is analyzed in step 818 and the process finishes in step 820. It is also possible to use this method with so called "reverse phase arrays" in which multiple samples are spotted on a slide, and then detected with labeled biospecifc binders, for example. The binders would be specific for molecules that are indicative of disease.

In the above cases, proteins are detected on a non-porous, or preferably, a porous substrate. As used herein a "non-porous substrate" is a substantially planar organic or inorganic solid surface on which the analytes reside. Non-porous substrates include, but are not limited to, glass slides, silicaceous slides and chips, such as silica or silicon-based materials and plastics, such as polycarbonate, acrylics, polystyrene, polyethylene, polypropylene and the like. As used herein a "porous substrate" is a substantially planar organic or inorganic porous surface on which the analytes reside. Porous substrates are generally membranes. Such membranes are well-known and can be made of nylons, PVDF and other well-known polymers.

In some cases, proteins can be detected directly in an electrophoresis gel, without transfer to a membrane. Typically, biological samples are homogenized by chemical or mechanical means and clarified by centrifugation. Soluble proteins are then separated on an electrophoresis gel. The separated proteins within the gel are then directly detected via their intrinsic Raman signal with antibodies conjugated with Raman labels. The gel is scanned using the inventive Raman spectrometer apparatus, signals from bound Raman-active probes are detected and amounts of respective proteins are calculated. Also, in some cases such as a solid bottom microtiter plate, analytes can be measured directly in solution.

It is also possible to perform indirect immunoassays in which the analyte is mixed with a labeled standard. The amount of free labeled standard is directly proportional to analyte concentration.

The inventive system can also be used to enhance other known techniques. In the detection and quantification of immunohistochemistry, tissues are typically thin sectioned, fixed on a glass slide and then stained with labeled antibodies directed against function/structure specific antigens. Stained sections are viewed under a microscope. The inventive system used with Raman dye labeled antibodies would allow quantification along with visualization. A similar result can be reached in detection and quantification of immunocytochemistry in which cultured cells are fixed and stained for cellular structure or specific proteins. The stained cells are then visualized with a microscope. In this case, Raman dye labeled antibodies, or another biospecific reagent, would allow quantification along with visualization.

The system is also useful in the detection and quantification of biomolecular interactions where one of the binding partners is immobilized. For study of biomolecular interactions and protein complexes, partners may be labeled with SERS tag or have specific intrinsic Raman signal. One of the interacting protein partners would be immobilized on a surface and would be identifiable through a Raman label or XY position. This surface would be contacted with a sample that contains a mix of proteins. Proteins that bind to the immobilized partner would be determined by their intrinsic signal. Alternatively, protein probes can be labeled with Raman-active dyes and their interaction with binding partners can be monitored by Raman detection. The surface is then scanned with the inventive Raman scanning spectrometer. This arrangement is useful for Protein-protein interactions where all partners in the interaction are proteins, Protein-nucleic acid interactions where one partner is a nucleic acid and another partner is a protein (more than one of each may participate in the complex), protein - small molecule interactions (proteins, drugs, etc.) where one partner is a small molecule and another partner is a protein (more than one of each may participate in the complex), Nucleic acid- nucleic acid interactions where all partners are nucleic acid molecules (both RNA and DNA molecules may participate in the interaction) and Nucleic acid-small molecule interactions where one partner is a small molecule and another partner is a nucleic acid. More than one of each may participate in the complex.

The inventive scanning apparatus is further useful in the detection and quantification of nucleic acids to include DNA and RNA. Typically, biological samples are homogenized by chemical or mechanical means and clarified by centrifugation. Nucleic acids are then separated on an electrophoresis gel. The separated nucleic acids within the gel are then transferred (blotted) to a porous membrane while maintaining their relative position. RNA or DNA probes labeled with Raman active dyes are used to probe the membrane in order to detect and quantify individual sequences. The SERS process allows for improved sensitivity, repeatability and accuracy. Such processes can include Northern and Southern blots, the detection and quantification of nucleic acids in a hydrogel the detection and quantification of nucleic acids in multi-well plates and on microarrays.

Also possible are the detection and identification of organisms through immunodetection of surface antigens by Raman labeled detection reagents or by intrinsic signal, including the detection and identification of prokaryotic organisms such as bacteria, viruses, mycoplasms. In this process organisms captured on a substrate (porous or non-porous) are probed with Raman dye labeled antibodies (or equivalent) directed against a surface antigen unique to an organism type. Scanning of that surface with the inventive Raman spectrometer would identify both the type and number of organisms.

Similarly, the apparatus can be used for the detection and identification of eukaryotic cells. Cells captured on a substrate (porous or non-porous) are probed with Raman labeled antibodies (or equivalent) directed against a surface antigen unique to a cell type. Scanning of that surface would identify both the type and number of cells. Further applications include detection and identification of organisms through nucleic acid hybridization with Raman labeled nucleic acid probes. For DNA hybridization assay, organisms on a substrate (porous or non-porous) are lysed releasing DNA. This DNA is then probed using a nucleic acid sequence unique to that organism that has been linked to a Raman label. Scanning of that surface would identify both the type and number of cells. For RNA hybridization assay, organisms on a substrate (porous or non-porous) are lysed releasing RNA. This RNA is then probed using a nucleic acid sequence unique to that organism that has been labeled with a Raman dye. Scanning of that surface would identify both the type and number of cells.

In another embodiment, latent fingerprints can be detected and characterized. The detected fingerprint shape is highly specific for individual identification. The latent fingerprint is first lifted from its original surface using a cellulosic or polymeric membrane, such as PVDF. A Raman label is then deposited onto the membrane. The deposition can be by immersion into a Raman label containing solution or deposition via a reagent delivery device, such as that disclosed in U.S. Patent Application Serial No. 12/145,018 filed on June 24, 2008 by L. Marco and T. Tague. The prepared fingerprint is then scanned with the Raman scanning device as described above. Characterization would consist of identification of residual materials or chemical compounds of interest, such as retained fibers, drugs, explosives, etc.

Those skilled in the art will know that the Raman signal detected by this scanner can be intrinsic, or imparted to one or more analytes through interaction with a detection reagent that contains a Raman label. There are a wide variety of chemical structures that generate a traceable Raman signal. They would also know that these signals are typically weak and thus may require some type of enhancement. Towards this end, the most common means of achieving such enhancement is to place the signal producing entity in close proximity to a noble metal configured as a film or nanoparticle. The process is commonly referred to as Surface Enhanced Raman Spectroscopy (SERS) and was first reported in 1974. See, "Raman Spectra of Pyridine Adsorbed at a Silver Electrode", M. Fleischmann, P.J. Hendra and A.J. McQuillan Chemical Physics Letters, v. 26, n. 2, pp.163-166 (15 May 1974).

Tarcha (US5376556 A) was amongst the first to demonstrate that SERS could be adapted for assays. The process described relies on diffusion to bring the enhancer in proximity to the Raman label. A further innovation to this application was described by Jing et al with aspects being incorporated into US20050089901 A1. See "Immunoassay Readout Method Using Extrinsic Raman Labels Adsorbed on Immunogold Colloids,", N. Jing, R. J. Lipert, G. B. Dawson and M. D. Porter, Analytical Chemistry, v. 71, n. 21, pp. 4903-4908 (1999). These publications teach the assembly of Extrinsic Raman labels (ERL) which are a pre-formed complex of biospecific binder, dye and nanoparticle. The complex fixes the key components together at an operable proximity removing the stochastic aspect of the earlier art which relied on diffusion.

US6514767 B1 and US7361410 B2 include additional examples of how such complexes can be constructed and there are other variants. For the purposes of this invention, all of these are considered a Raman label or Raman labeled detection reagent.

The use of SERS label conjugated secondary antibodies eliminates many of the problems encountered with conventional Western blot and ELISA protein detection. For example, with SERS labels, the detected signal is produced directly from the SERS dye and not indirectly from an enzymatic reaction. The SERS labels do not require further blot handling as with ECL and colorimetry detection; nor are they hazardous as is the case with radioactive tags. Significantly, there is more than one type of SERS dye and each dye or label emits a separate and distinguishable signal. This provides the ability to perform multiplex analysis on a single blot, increasing the information content while reducing handling and processing time. Together, these features impart greater sensitivity, reduced variability, reduced handling time, and greater information content compared to existing technologies.

In some cases fluorescence of the Raman label may be used for high concentration detection in order to improve the dynamic range of the system. The aforementioned SERS method provides the best sensitivity, but can be saturated for high protein content. In this case the SERS data is collected first, followed by a fluorescence scan only if saturation is observed.

While the invention has been shown and described with reference to a number of embodiments thereof, it will be recognized by those skilled in the art that various changes in form and detail may be made herein without departing from the scope of the invention as defined by the appended claims.

## Claims

1. A Raman spectral mapping spectrometer (100) having a line-shaped illumination pattern (107) and a translation stage (118) for generating a Raman spectral map of a sample (114), the spectrometer (100) comprising:
an excitation source that generates an excitation beam; a spectral analyzer having an entrance slit (136);
an optical system that focuses the excitation beam onto the sample in a line-shaped illumination pattern thereby generating a Raman signal and focuses the Raman signal on the entrance slit;
and a translation stage (118) that moves the sample (114) continuously, during the entire acquisition period of the detector (128) for each spectrum, in a direction that is perpendicular to the line-shaped illumination pattern (107), so that a spectrum is obtained over a rectangular area, whose sides are defined by the line- shaped illumination pattern and the length the stage travelled during the spectral acquisition period;
and a computer (130) for controlling the stage (118) and the spectral acquisition time of the detector (128) and the stage travel speed, so as to control the spatial resolution of the spectral map in the stage travel direction, with said spatial resolution equal to the product of the spectral acquisition time and the stage travel speed.

2. The device of claim 1, further comprising a multi-channel detector.

3. The device of claim 1 or claim 2 wherein the optical system comprises an objective lens for focusing the excitation beam on the sample, beam-combining optics for directing the excitation beam to the objective lens and at least one optical device located between the excitation source and the beam combining optics that forms the line-shaped illumination pattern on the sample.

4. The device of claim 1 or claim 2, wherein the optical system comprises an objective lens for focusing the excitation beam on the sample, beam-combining optics for directing the excitation beam to the objective lens and at least one optical device located between the beam combining optics and the objective lens that forms the line-shaped illumination pattern on the sample.

5. The device of claim 3 or claim 4, wherein the optical device comprises at least one of the group consisting of a cylindrical lens, a Powell lens and a scanning optic.

6. The device of claim 5, wherein the scanning optic comprises a Galvano mirror.

7. The device of claim 6 wherein the Galvano mirror is located between the sample and the entrance slit so that the Galvano mirror scans the Raman signal on the entrance slit.

8. A method for detecting and quantifying analytes by Surface Enhanced Raman scattering (SERS) with a spectrometer generating a two dimensional Raman spectral map of a sample according to anyone of the preceding claims, the method comprising:
depositing the analytes on a substrate and
(a) using a separation process to physically separate the analytes on a substrate and transferring the separated analytes from the substrate onto a surface;
(b) contacting the surface with a detection reagent containing antibodies that selectively bind to the analytes and that are labeled with Raman active labels;
(c) scanning the surface with a laser to generate a SERS signal; and
(d) detecting and analyzing the SERS signal based on physical positioning on the surface to detect and quantify the analytes;
further comprising:
(c1) projecting on the sample an excitation beam having a line-shaped illumination pattern having a line length;
(c2) physically translating the sample in a direction perpendicular to the line length; and
(d1) acquiring a Raman signal generated from the sample in response to the excitation beam as the sample is moving so that the map is generated pixel by pixel, wherein each pixel represents a rectangular area of the sample, the area having a width equal to the line length and a length equal to a distance traversed by the sample during a predetermined time,
wherein step (c) comprises positioning the line-shaped illumination pattern on one side of the rectangular area, accumulating the Raman signal on a detector while translating the sample in a direction perpendicular to the line-shaped illumination pattern, and generating a Raman spectrum when the line-shaped illumination pattern reaches an opposing side of the rectangular area.

9. The method of claim 8 wherein step (a) comprises physically separating the analytes within a gel by electrophoresis and blotting the gel to transfer the analytes onto the surface.

10. The method of claim 8 or claim 9, wherein the sample is a Western blot having a plurality of parallel lanes extending in a same direction and wherein step (c2) comprises translating the sample so that a plurality of the lanes are traversed in a raster pattern.

## Patentansprüche

1. Raman-Spektrometer (100) für Spectral Mapping mit einem linienförmigen Beleuchtungsmuster (107) und einem Verschiebetisch (118) zum Erzeugen einer Raman-Spektralabbildung einer Probe (114), wobei das Spektrometer (100) aufweist:
eine Anregungsquelle, die einen Anregungsstrahl erzeugt; einen Spektralanalysator mit einem Eintrittsspalt (136);
ein optisches System, das den Anregungsstrahl in einem linienförmigen Beleuchtungsmuster auf die Probe fokussiert und dadurch ein Ramansignal erzeugt und das Ramansignal auf den Eintrittsspalt fokussiert;
und einen Verschiebetisch (118), der die Probe (114) während der gesamten Erfassungsperiode des Detektors (128) für jedes Spektrum kontinuierlich in eine Richtung senkrecht zu dem linienförmigen Beleuchtungsmuster (107) bewegt, so dass ein Spektrum über einen rechteckigen Bereich erhalten wird, dessen Seiten durch das linienförmige Beleuchtungsmuster und die Strecke, die der Verschiebetisch während der Spektrum-Erfassungsperiode zurückgelegt hat, definiert sind;
und einen Computer (130) zum Steuern des Verschiebetischs (118) und der Spektrum-Erfassungszeit des Detektors (128) und der Bewegungsgeschwindigkeit des Verschiebetischs, um die räumliche Auflösung der Spektralabbildung in der Bewegungsrichtung des Verschiebetischs zu steuern, wobei die räumliche Auflösung gleich dem Produkt aus der Spektrum-Erfassungszeit und der Bewegungsgeschwindigkeit des Verschiebetischs ist.

2. Vorrichtung nach Anspruch 1, weiterhin umfassend einen Multi-Kanal-Detektor.

3. Vorrichtung nach Anspruch 1 oder 2, wobei das optische System aufweist: eine Objektivlinse zum Fokussieren des Anregungsstrahls auf die Probe, eine Strahlen vereinigende Optik zum Richten des Anregungsstrahls auf die Objektivlinse und mindestens eine optische Vorrichtung, die zwischen der Anregungsquelle und der Strahlen vereinigenden Optik, die das linienförmige Beleuchtungsmuster auf der Probe bildet, angeordnet ist.

4. Vorrichtung nach Anspruch 1 oder 2, wobei das optische System aufweist: eine Objektivlinse zum Fokussieren des Anregungsstrahls auf die Probe, eine Strahlen vereinigende Optik zum Richten des Anregungsstrahls auf die Objektivlinse und mindestens eine optische Vorrichtung, die zwischen der Strahlen vereinigenden Optik und der Objektivlinse, die das linienförmige Beleuchtungsmuster auf der Probe bildet, angeordnet ist.

5. Vorrichtung nach Anspruch 3 oder 4, wobei die optische Vorrichtung mindestens eines aus der Gruppe bestehend aus zylindrischer Linse, einer Powell-Linse und einer Scanoptik, aufweist.

6. Vorrichtung nach Anspruch 5, wobei die Scanoptik einen Galvanospiegel aufweist.

7. Vorrichtung nach Anspruch 6, wobei der Galvanospiegel zwischen der Probe und dem Eintrittsspalt angeordnet ist, so dass der Galvanospiegel das Ramansignal an dem Eintrittsspalt scannt.

8. Verfahren zum Detektieren und Quantifizieren von Analyten durch oberflächenverstärkte Raman-Streuung (Surface Enhanced Raman Scattering SERS) mit einem Spektrometer, das eine zweidimensionale Raman-Spektralabbildung einer Probe nach einem der vorhergehenden Ansprüche erzeugt, wobei das Verfahren umfasst:
Aufbringen der Analyten auf ein Substrat und
(a) Verwenden eines Trennvorgangs, um die Analyten auf einem Substrat physisch zu trennen und die getrennten Analyten von dem Substrat auf eine Fläche zu übertragen;
(b) In Kontakt bringen der Fläche mit einem Detektionsreagenz, das Antikörper enthält, die sich selektiv an die Analyten binden und die mit Raman-aktiven Markierungen markiert sind;
(c) Scannen der Fläche mit einem Laser zum Erzeugen eines SERS-Signals; und
(d) Detektieren und Analysieren des SERS-Signals auf der Basis der physischen Positionierung auf der Fläche, um die Analyten zu detektieren und zu quantifizieren; weiterhin umfassend:
(c1) Projizieren eines Anregungsstrahls mit einem linienförmigen Beleuchtungsmuster mit einer Linienlänge auf die Probe;
(c2) Physische Umsetzung der Probe in eine Richtung senkrecht zur Linienlänge; und
(d1) Erfassen eines Ramansignals, das von der Probe in Reaktion auf den Anregungsstrahl erzeugt wird, während die Probe sich bewegt, so dass die Abbildung Pixel um Pixel erzeugt wird, wobei jedes Pixel einen rechteckigen Bereich der Probe darstellt, wobei der Bereich eine Breite hat, die der Linienlänge entspricht, und eine Länge hat, die gleich einem Abstand ist, den die Probe während einer vorbestimmten Zeit durchläuft,
wobei Schritt (c) umfasst: Positionieren des linienförmigen Beleuchtungsmusters an einer Seite des rechteckigen Bereichs, Akkumulieren des Ramansignals an einem Detektor, während die Probe in eine Richtung senkrecht zu dem linienförmigen Beleuchtungsmuster umgesetzt wird, und Erzeugen eines Ramanspektrums, wenn das linienförmige Beleuchtungsmuster eine gegenüberliegende Seite des rechteckigen Bereichs erreicht.

9. Verfahren nach Anspruch 8, wobei Schritt (a) das physische Trennen der Analyten innerhalb eines Gels durch Elektrophorese und Blotten des Gels zum Übertragen der Analyten auf die Fläche umfasst.

10. Verfahren nach Anspruch 8 oder 9, wobei die Probe ein Western Blot mit einer Vielzahl von parallelen Spuren ist, die sich in dieselbe Richtung erstrecken und wobei Schritt (c2) das Umsetzen der Probe umfasst, so dass eine Vielzahl der Spuren in einem Rastermuster durchlaufen werden.

## Revendications

1. Spectromètre de mappage spectral de Raman (100) ayant un motif d'illumination en forme de ligne (107) et une platine de translation (118) pour générer une carte spectrale de Raman d'un échantillon (114), le spectromètre (100) comprenant :
une source d'excitation qui génère un faisceau d'excitation, un analyseur spectral ayant une fente d'entrée (136),
un système optique qui focalise le faisceau d'excitation sur l'échantillon suivant un motif d'illumination en forme de ligne en générant de cette manière un signal de Raman et focalise le signal de Raman sur la fente d'entrée,
et une platine de translation (118) qui déplace l'échantillon (114) de manière continue, durant la période d'acquisition entière du détecteur (128) pour chaque spectre, dans une direction qui est perpendiculaire au motif d'illumination en forme de ligne (107), de sorte qu'un spectre soit obtenu sur une zone rectangulaire, dont les côtés sont définis par le motif d'illumination en forme de ligne et la longueur de la platine parcourue au cours de la période d'acquisition spectrale,
et un ordinateur (130) destiné à commander la platine (118) et le temps d'acquisition spectrale du détecteur (128) et la vitesse de déplacement de platine, de manière à commander la résolution spatiale de la carte spectrale dans la direction de déplacement de platine, ladite résolution spatiale étant égale au produit du temps d'acquisition spectrale et de la vitesse de déplacement de platine.

2. Dispositif selon la revendication 1, comprenant en outre un détecteur à plusieurs canaux.

3. Dispositif selon la revendication 1 ou la revendication 2 dans lequel le système optique comprend un objectif destiné à focaliser le faisceau d'excitation sur l'échantillon, une optique de combinaison de faisceau destinée à diriger le faisceau d'excitation vers l'objectif et au moins un dispositif optique situé entre la source d'excitation et l'optique de combinaison de faisceau qui forme le motif d'illumination en forme de ligne sur l'échantillon.

4. Dispositif selon la revendication 1 ou la revendication 2, dans lequel le système optique comprend un objectif destiné à focaliser le faisceau d'excitation sur l'échantillon, une optique de combinaison de faisceau destinée à diriger le faisceau d'excitation vers l'objectif et au moins un dispositif optique situé entre l'optique de combinaison de faisceau et l'objectif qui forme le motif d'illumination en forme de ligne sur l'échantillon.

5. Dispositif selon la revendication 3 ou la revendication 4, dans lequel le dispositif optique comprend au moins un élément d'un groupe constitué d'une lentille cylindrique, d'une lentille de Powell et d'une optique de balayage.

6. Dispositif selon la revendication 5, dans lequel l'optique de balayage comprend un miroir Galvano.

7. Dispositif selon la revendication 6 dans lequel le miroir Galvano est situé entre l'échantillon et la fente d'entrée de sorte que le miroir Galvano balaye le signal de Raman sur la fente d'entrée.

8. Procédé de détection et de quantification d'analytes par diffusion de Raman exaltée par effet de surface (SERS) avec un spectromètre générant une carte spectrale de Raman à deux dimensions d'un échantillon selon l'une quelconque des revendications précédentes, le procédé comprenant les étapes consistant à :
déposer les analytes sur un substrat et
(a) utiliser un procédé de séparation pour séparer physiquement les analytes sur un substrat et transférer les analytes séparés à partir du substrat sur une surface,
(b) mettre en contact la surface avec un réactif de détection contenant des anticorps qui se lient sélectivement aux analytes et qui sont repérés avec des marqueurs actifs de Raman,
(c) balayer la surface avec un laser pour générer un signal SERS, et
(d) détecter et analyser le signal SERS à partir d'un positionnement physique sur la surface pour détecter et quantifier les analytes,
comprenant en outre les étapes consistant à :
(c1) projeter sur l'échantillon un faisceau d'excitation ayant un motif d'illumination en forme de ligne ayant une longueur de ligne,
(c2) faire translater physiquement l'échantillon dans une direction perpendiculaire à la longueur de ligne, et
(d1) acquérir un signal de Raman généré à partir de l'échantillon en réponse au faisceau d'excitation à mesure que l'échantillon se déplace de sorte que la carte soit générée pixel par pixel, dans lequel chaque pixel représente une zone rectangulaire de l'échantillon, la zone ayant une largeur égale à la longueur de ligne et une longueur égale à une distance traversée par l'échantillon sur une durée prédéterminée,
dans lequel l'étape (c) comprend le positionnement du motif d'illumination en forme de ligne sur un premier côté de la zone rectangulaire, l'accumulation du signal de Raman sur un détecteur tout en faisant translater l'échantillon dans une direction perpendiculaire au motif d'illumination en forme de ligne, et la génération d'un spectre de Raman lorsque le motif d'illumination en forme de ligne atteint un côté opposé de la zone rectangulaire.

9. Procédé selon la revendication 8 dans lequel l'étape (a) comprend la séparation physique des analytes dans un gel par électrophorèse et buvardage du gel pour transférer les analytes sur la surface.

10. Procédé selon la revendication 8 ou la revendication 9, dans lequel l'échantillon est un transfert de Western ayant une pluralité de couloirs parallèles s'étendant dans une même direction et dans lequel l'étape (c2) comprend la translation de l'échantillon de sorte qu'une pluralité des couloirs soient traversés dans un motif de trame.
